# EUROPEAN PATENT APPLICATION

(11) **EP 1 374 938 A1**
(43) Date of publication of application: **02.01.2004**
(21) Application number: 03445078.3
(22) Date of filing: 18.06.2003
(51) Int. Cl.: A61M 16/00

(54) **Medical ventilator**

(30) Priority: 18.06.2002 SE 0201854
(71) Applicant: Siemens-Elema AB, 171 95 Solna (SE)
(72) Inventor: Dan Lindén, SE, 115 43 Stockholm (SE)

(57) **Abstract**

A medical ventilator (2) comprises a user interface (16) with a screen (18) adapted to display curves (28) representing function parameters for the medical ventilator (2), a memory unit (22) for storing target values for the function parameters, and a control unit (14) for controlling the medical ventilator (2) dependent on the target values for the function parameters stored in the memory unit (22) is disclosed. A simplified method of modifying or programming the medical ventilator is achieved through adaptation of the user interface (16) to register a new curve and/or changes to a displayed curve (28) performed by a user on the screen (18) and to transfer these to the memory unit (22) for storage as new target values for the function parameters.

## Description

The present invention relates to a medical ventilator according to the preamble of Claim 1.

The user interface is an important component of a medical ventilator. It normally includes a screen that can be used to display numerical and graphical information related to operating parameters, ventilation modes, monitored parameters, respiration curves, etc. One such interface is described in US 5,881,723.

It is also known to provide a medical ventilator with a user interface having an interactive screen. An example of such is the Servoⁱ® ventilator from Siemens Elema AB, Sweden. This user interface comprises an interactive screen that selectively can be used for programming of functions and as a monitor to display breathing curves and other information.

In the present context programming of functions means chiefly breathing modes, where the parameter values can be input and numerically displayed on a screen.

In certain situations it would be desirable to input or modify a breathing mode outside the normal input possibilities. This should be possible to perform in a simple and user-friendly fashion, while still being accurate and precise.

US 5,931,160 describes a control system for ventilators, which allows the essentially free setting of different breathing parameters. The setting is achieved via usual installation methods. The input values can possibly be displayed on a screen. This programming requires the user to input a whole range of parameters, successively leading to a ventilation mode.

An aim of the present invention is to create a medical ventilator according to the above which at least partly addresses the above stated problems and desires.

Such a medical ventilator is achieved according to the invention by means of the medical ventilator being devised as is evident in the characterising portion of Claim 1.

Advantageous refinements and embodiments are set out in the claims dependent on Claim 1.

With a user interface, designed to register desired settings or changes to settings, a very simple and direct work tool is obtained for the operator. No buttons need be pressed. A new curve shape, for example the pressure, can quickly and simply be drawn in by the operator and implemented by the medical ventilator.

To avoid unintentional changes of the installed values the interactive screen can be designed so that a switch must be adjusted before changes can be implemented.

Restoration of the last (fixed) installed operational mode can be done by means of another switch.

Further advantages/details of the medical ventilator according to the invention are set out in the following description of an exemplary embodiment of the medical ventilator according to the invention, in which:
FIG. 1 shows a schematic representation of an exemplary embodiment of a medical ventilator according to the invention;
FIG. 2 shows an interactive screen in the medical ventilator according to FIG. 1; and
FIG. 3 shows an alternative arrangement of a screen in the medical ventilator according to the invention.

With reference to FIG: 1, an exemplary embodiment of a medical ventilator 2 according to the invention is shown. The medical ventilator 2 comprises a pneumatic unit 4 for the preparation of a breathing gas. In this present case the pneumatic unit 4 has two gas inlets 6A, 6B for the coupling in of two gases, for example oxygen and air.

The prepared breathing gas is carried towards a patient 8 via an inspiration line 10 during inspiration and away from the patient 8 via an expiration line 12 during expiration.

The medical ventilator 2 further comprises a control unit 14 for regulation and control of the pneumatic unit 4 and a user interface 16, through which an operator can install a suitable ventilation mode for the patient 8.

The user interface 16 comprises in this embodiment an interactive screen 18. To increase safety against unwanted changes or settings a first function switch 20A may be included. Interactive measures between the operator and screen 18 would the only be permitted after activation of the first function switch 20A.

To facilitate the resetting of the last programmed setting the user interface 16 may include a second function switch 20B. Upon activation of the second function switch 20B last used setting (for controlling supply of gas to a patient) is restored.

The function of switches 20A, 20B can of course be implemented in a single switch. The switches 20A, 20B can be hard ware switches located next to or on the interactive screen 18, soft ware based switches displayed on the screen, or a mixture of the two.

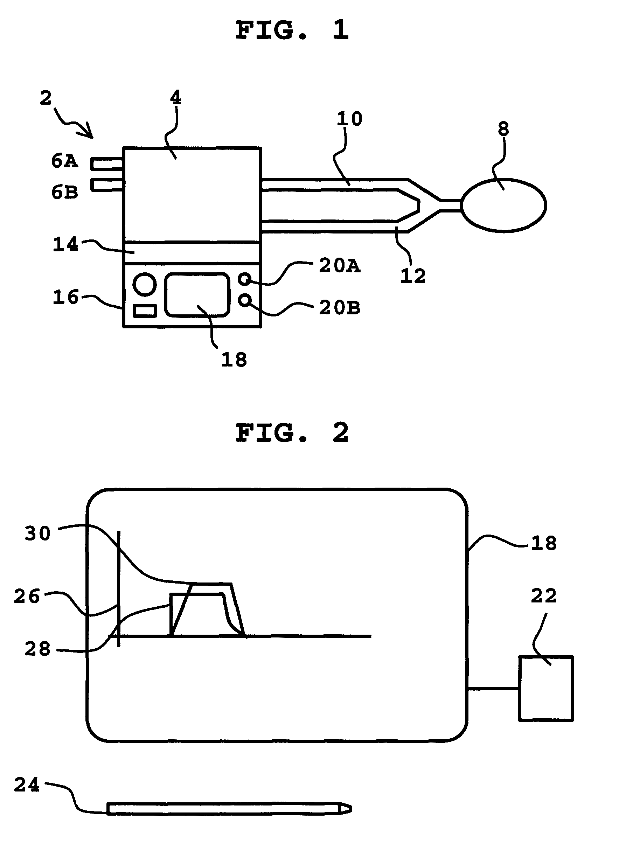

In FIG. 2 the interactive screen 18 is shown more clearly. A memory unit 22, which is here connected to the screen 18, is also shown. The memory unit 22 is also connected to the control unit 14 in FIG. 1 (not shown in the figure).

The function parameters for the ventilation mode which shall be applied to the patient 8 are found in the memory unit 22. More specifically there is to be found, amongst other things, target values for one or some of the parameters: pressure, flow, inspiration time and expiration time. Other parameters may also be found, such as composition of the breathing gas, etc.

The interactive screen 18 of the foregoing exemplary embodiment can be modified by means of a pointer device 24. The pointer device 24 is not essential but does allow a more precise revision of the screen contents than does the use of a finger.

A coordinate system 26 is drawn on the screen 18 as an example of a graphic representation of the actual ventilation mode (corresponding target values in memory unit 22). A first curve 28 is displayed in the coordinate system 26, which corresponds to a pre-programmed working mode (pressure on the vertical axis and time on the horizontal axis) over a breathing cycle (inspiration and expiration).

In accordance with the present invention the operator can modify the existing ventilation mode (alternatively create his own) by changing the first curve 28 to the second curve 30 directly on the screen 18. Values for the second curve 30 are then registered in the memory unit 22 as target values and transferred to the control unit 14 for the control of the pneumatic unit 4 according to the modified ventilation mode.

In order to facilitate the setting for the user, a numeric value could be displayed indicating the value of the touched point on the screen. The value could be indicated in a box that follows the movements made by the user on the screen.

The new input value can be implemented as the pointer device 24 is lifted from the screen or via a confirmation step.

If an entirely own ventilation mode is to be created the coordinate system can be blank.

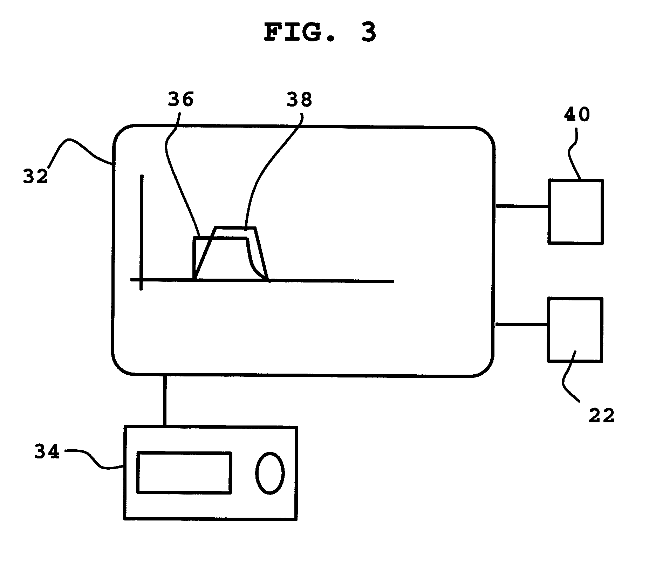

FIG. 3 shows another embodiment with a screen 32. The screen 32 is not interactive and inputs are made through a user console 34, which could be a keyboard, mouse or any other device for entering data (alone or in combination). A mouse or similar device may be preferable as they provide a more easily movable cursor or indicator on the screen. All known means of transferring the information (wire, wire-less) can be utilised.

The purpose is still to create a curve on the screen 32, either by drawing the entire curve or supplementing key points that are then combined into a curve by the user interface (interpolation).

As an alternative to going from a blank screen or from target values to an existing programmed ventilation mode, a measured curve 36 can be shown on the screen 18 and modified in a corresponding way to a modified curve 38. The measured curve can be taken from a monitoring unit 40. This essentially means that a measured curve 36 showing what happens near or in the lungs of a patient can be used as basis for modifications of a treatment.

Thereby the intended or desired effect of a certain ventilation mode can be achieved in an even more effective manner. The registration of curves for pressure, flow and volume is made, as a rule, in the majority of medical ventilators. In the event of a certain parameter or variable being missing from the medical ventilator's normal monitoring features then a corresponding separate parameter meter can simply be connected in order to attain a relevant measurement signal.

The actual changes to/insertions of curves on the screen 32 can be achieved in many different ways as understood from the above description in relation to FIG. 2 and FIG. 3. One way is for the operator to draw in the complete curve and another is for the operator to insert key points, after which the user interface adapts a curve to the inserted points.

Naturally, combinations in functionality and hard ware/soft ware can be made between the two embodiments of FIG. 2 and FIG. 3. Typically, monitored curves as shown in FIG. 3 could also be utilised in the embodiment of FIG. 2.

Further safety functions can, of course, also be utilised for the disclosed manner of input. There will then be no risk for the patient of being exposed to too great pressures and/or volumes, unreasonably short or long inspiration and expiration times, etc. Basically, any known safety function relating to the overall operation of the medical ventilator vis-à-vis the patient can be present.

All breathing apparatus for medical use are included in the context of medical ventilator used in the present application. Accordingly, respirators or ventilators for intensive care, anaesthetic apparatus, respirators or ventilators for sub-acute, respirators for home care, etc., are included.

## Claims

1. A medical ventilator (2) comprising a user interface (16) with a screen (18) adapted to display curves (28) representing function parameters for the medical ventilator (2), a memory unit (22) for storing target values for the function parameters, and a control unit (14) for controlling the medical ventilator (2) dependent on the target values for the function parameters stored in the memory unit (22), **characterised in that** the user interface (16) is adapted to register a new curve and/or changes to a displayed curve (28) performed by a user on the screen (18) and transfer these to the memory unit (22) for storage as new target values for the function parameters.

2. A medical ventilator according to claim 1, **characterised in that** the user interface (16) is adapted to register a new curve and/or changes to a displayed curve (28) by interpolation between points on the screen input by the user.

3. A medical ventilator according to claim 1 or 2, **characterised in that** the user interface (16) comprises a first function switch (20A) whereby registration of the new curve and/or changes to the displayed curve (28) performed by the user on the screen (18) can only be made after activation of the first function switch (20A).

4. A medical ventilator according to claim 3, **characterised in that** the user interface (16) comprises a second function switch (20B) and **in that** the memory unit (22) is adapted to recover earlier stored target values on activation of the second function switch (20B).

5. A medical ventilator according to any of the preceding claims, **characterised in that** the screen (18) is an interactive screen.

6. A medical ventilator according to claim 5, **characterised in that** there is provided a stand-alone pointer device (24) by which the user can perform the changes to the displayed curves (28) on the interactive screen (18).
